(19) Europäisches Patentamt
European Patent Office
Office européen des brevets

(11) **EP 3 588 067 B1**

(12) **FASCICULE DE BREVET EUROPEEN**

(45) Date de publication et mention
de la délivrance du brevet:
**06.01.2021 Bulletin 2021/01**

(51) Int Cl.:
***G01N 23/087*** *(2018.01)*     ***A61B 6/00*** *(2006.01)*

(21) Numéro de dépôt: **19181552.1**

(22) Date de dépôt: **20.06.2019**

(54) **PROCÉDÉ DE CARACTÉRISATION D'UN OBJET PAR IMAGERIE SPECTRALE**

CHARAKTERISIERUNGSVERFAHREN EINES OBJEKTS DURCH
SPEKTRALBILDGEBUNGSVERFAHREN

METHOD FOR CHARACTERISING AN OBJECT BY SPECTRAL IMAGING

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **22.06.2018 FR 1855616**

(43) Date de publication de la demande:
**01.01.2020 Bulletin 2020/01**

(73) Titulaire: **Commissariat à l'Énergie Atomique
et aux Énergies Alternatives
75015 Paris cedex (FR)**

(72) Inventeurs:
• **PAULUS, Caroline
38054 Grenoble cedex 09 (FR)**
• **BRAMBILLA, Andréa
38054 Grenoble cedex 09 (FR)**
• **REBUFFEL, Veronique
38054 Grenoble Cedex 09 (FR)**

(74) Mandataire: **INNOV-GROUP
310, avenue Berthelot
69372 Lyon Cedex 08 (FR)**

(56) Documents cités:
**EP-A1- 3 106 864      WO-A1-2015/091083
WO-A1-2017/211625      GB-A- 2 454 782**

• **ALVAREZ ROBERT: "Near optimal energy
selective x-ray imaging system performance with
simple detectors", MEDICAL PHYSICS, AIP,
MELVILLE, NY, US, vol. 37, no. 2, 27 janvier 2010
(2010-01-27), pages 822-841, XP012135607, ISSN:
0094-2405, DOI: 10.1118/1.3284538**

**Description**

**DOMAINE TECHNIQUE**

Le domaine technique de l'invention est la caractérisation d'un objet par irradiation à l'aide d'une source d'irradiation X ou gamma et la formation d'une image spectrale d'un rayonnement transmis par l'objet.

**ART ANTERIEUR**

La caractérisation d'objets par irradiation à l'aide d'un rayonnement électromagnétique ionisant, par exemple un rayonnement X ou gamma, permet d'estimer la nature des matériaux composant cet objet. Il est usuel de former une image représentative de l'atténuation du rayonnement par l'objet. Cette image permet d'effectuer une caractérisation bidimensionnelle.

Une telle caractérisation peut être réalisée à des fins de diagnostic médical, de contrôle non destructif dans le domaine industriel, ou encore dans la détection de matériaux dangereux ou illicites, par exemple dans des portiques de contrôle d'individus, de type portiques d'aéroport, ou dans le contrôle de bagages.

L'émergence de détecteurs spatialement et spectralement résolus a permis d'améliorer sensiblement les performances de ces caractérisations. Ce type de détecteur permet de former une image spectrale de l'atténuation de l'objet analysé. Par image spectrale, on entend une image dans différents canaux d'énergie. Une image spectrale comporte différents pixels. A chaque pixel est associé un spectre du rayonnement détecté par le détecteur. Ce spectre comporte plusieurs canaux, chaque canal étant représentatif d'une bande d'énergie. Un tel spectre peut comporter des dizaines voire des centaines de canaux.

La publication Alvarez R "Near optimal energy selective x-ray imaging system performance with simple detectors", Med. Phys. 37 (2), February 2010 décrit une caractérisation d'un objet par formation d'un spectre représentant une atténuation d'un rayonnement ionisant par l'objet. Cette publication décrit une décomposition de l'atténuation, de façon linéaire, dans une base composée de l'atténuation de matériaux connus. Par atténuation d'un objet, il est entendu l'atténuation d'un rayonnement, tel que précédemment défini, par l'objet. Par exemple, lorsqu'on dispose d'un objet 20 à caractériser, la fonction d'atténuation spectrale $att_{20}$ de l'objet analysé peut être approximée par une combinaison linéaire de fonctions d'atténuation spectrale de matériaux connus $mat1$ et $mat2$ selon l'expression suivante :

$$att_{20} \approx L_1\mu_1 + L_2\mu_2 \ (1)$$

où :

- $att_{20}$ est l'atténuation spectrale de l'objet examiné. Le terme "spectrale" désigne le fait qu'il s'agit d'une fonction définie à différentes l'énergies E. L'atténuation est proportionnelle au logarithme du spectre $S$ du rayonnement, atténué par l'objet, et détecté par le détecteur, selon l'expression $att_{20} \ \alpha$ -ln($S$) où $\alpha$ désigne la proportionnalité.
- $\mu_1$ et $\mu_2$ sont respectivement les fonctions spectrales d'atténuation linéique du premier matériau et du deuxième matériau. Par atténuation linéique, on entend une atténuation par unité de longueur.
- $L_1$ et $L_2$ sont des épaisseurs, dites épaisseurs équivalentes, du premier matériau $mat1$ et du deuxième matériau $mat2.$
- $\approx$ signifie "étant approximé par".

L'atténuation spectrale de l'objet $att_{20}$ est obtenue à partir du spectre du rayonnement détecté par le détecteur, $att_{20} \approx -ln\left(\frac{S}{S_o}\right),$ $S_o$ étant un spectre du rayonnement détecté par le détecteur en l'absence d'objet disposé entre la source d'irradiation et le détecteur.

A partir de la mesure du spectre S, l'objet peut être caractérisé par une estimation des épaisseurs équivalentes $L_1$ et $L_2$. L'intérêt de disposer d'une information spectrale de l'atténuation d'un objet, selon au moins deux canaux d'énergie, permet de déterminer les épaisseurs équivalentes $L_1$ et $L_2$. Pour cela, l'atténuation $att_{20}$ de l'objet doit être mesurée selon au moins deux canaux d'énergie. Le document EP3106864 propose une méthode d'estimation des épaisseurs équivalentes $L_1$ et $L_2$ basée sur une approche de type maximum de vraisemblance.

En formant une image spectrale de l'objet, c'est un dire une distribution spatiale de spectres du rayonnement atténué par l'objet, on peut obtenir des épaisseurs équivalentes selon différents pixels, chaque pixel correspondant à une partie de l'objet.

Les inventeurs ont souhaité apporter un perfectionnement aux méthodes existantes, qui permet une caractérisation d'un objet par une détermination d'épaisseurs équivalentes en nécessitant une moindre irradiation de l'objet examiné.

## EXPOSE DE L'INVENTION

Un premier objet de l'invention est un procédé de caractérisation d'un objet comportant les étapes suivantes :

a) disposition de l'objet entre une source d'irradiation et un détecteur de rayonnement, la source d'irradiation étant configurée pour émettre un rayonnement électromagnétique ionisant se propageant jusqu'à l'objet ;
b) irradiation de l'objet par la source d'irradiation et détection d'un rayonnement transmis par l'objet à l'aide du détecteur de rayonnement, le détecteur de rayonnement comportant plusieurs pixels, chaque pixel étant associé à une partie de l'objet ;
c) pour chaque pixel, formation d'un spectre d'énergie du rayonnement détecté, chaque spectre comportant au moins deux bandes d'énergie distinctes ;
d) à partir de chaque spectre formé lors de l'étape c), estimation, en chaque pixel, d'au moins deux épaisseurs équivalentes respectivement associées à au moins deux matériaux de base;

le procédé étant caractérisé en ce qu'il comporte, suite à l'étape d), les étapes suivantes :

e) à partir des épaisseurs équivalentes résultant de l'étape d), calcul d'un paramètre structurel de l'objet en différents pixels;
f) lissage spatial du paramètre structurel calculé en plusieurs pixels, de façon à associer, à chaque pixel, un paramètre structurel lissé;
g) à partir du paramètre structurel lissé en chaque pixel, et à partir de chaque spectre formé lors de l'étape c), estimation, en chaque pixel, d'épaisseurs équivalentes régularisées respectivement associées à chaque matériau de base ;
h) caractérisation de l'objet à partir des épaisseurs équivalentes régularisées estimées lors de l'étape g).

A chaque pixel est associée une partie de l'objet, cette dernière étant vue par le pixel, c'est-à-dire disposée dans un angle solide sous lequel le pixel voit l'objet. Par paramètre structurel de l'objet en un pixel, on entend un paramètre structurel de la partie de l'objet associée au pixel. De même, par épaisseur équivalente en un pixel, on entend l'épaisseur équivalente de la partie de l'objet associée au pixel.
Selon un mode de réalisation, l'étape d) et/ou l'étape g) comporte, pour chaque pixel, une prise en compte de spectres de calibration, chaque spectre de calibration étant associé à une épaisseur de chaque matériau de base. L'étape d) et/ou l'étape g) peut également comporter également, pour chaque pixel:

- un calcul d'une fonction de vraisemblance à partir du spectre mesuré par le pixel lors de l'étape c) et à partir de spectres de calibration, chaque spectre de calibration étant associé à au moins un matériau de calibration d'une épaisseur connue ;
- une détermination d'une épaisseur équivalente de chaque matériau de calibration maximisant la fonction de vraisemblance, chaque matériau de calibration formant un matériau de base.

Un spectre de calibration peut être un spectre mesuré ou modélisé en remplaçant l'objet par un objet de calibration, l'objet de calibration étant formé d'une épaisseur d'au moins un matériau de calibration. Le procédé suppose le recours à différents spectres de calibration, correspondant respectivement à des épaisseurs différentes d'au moins deux matériaux de calibration différents. Les matériaux de calibration peuvent correspondre aux matériaux de base.
Les étapes d) et g) peuvent comporter une étape de changement de base, entre une base de départ, formé par les matériaux de calibration, et une base d'arrivée, formée par des matériaux de base représentatifs de l'objet, ou considérés comme représentatifs de l'objet, de façon à obtenir une épaisseur équivalente de chaque matériau de la base d'arrivée. Ainsi, à partir d'épaisseurs équivalentes de chaque matériau de calibration, formant la base de départ, on obtient une épaisseur équivalente de chaque matériau formant la base d'arrivée. Les matériaux formant la base d'arrivée peuvent par exemple être des matériaux physiologiques, par exemple un tissu, ou un certain type de tissu, ou os. Le changement de base peut être établi en prenant en compte une matrice de changement de base.
L'étape d) peut comporter :

- un regroupement de pixels adjacents, pour former un groupe de pixels;
- une association, à chaque groupe de pixels, d'un spectre regroupé, combinant les spectres formés pour chaque pixel du groupe de pixels ;

de telle sorte que l'étape d) est mise en oeuvre, pour au moins un pixel d'un groupe de pixels, à partir du spectre regroupé associé au groupe de pixels.

Lors de l'étape e), pour chaque pixel, le paramètre structurel peut être une épaisseur de la partie de l'objet associée au pixel.

Lors de l'étape e), pour chaque pixel, le paramètre structurel peut également représenter une composition de la partie de l'objet associée au pixel. Dans ce cas, le paramètre structurel, déterminé en chaque pixel, peut :

- être un numéro atomique effectif, déterminé à partir des épaisseurs équivalentes estimées lors de l'étape d) ;
- ou comporter un ratio entre une épaisseur équivalente d'un matériau de base sur la somme des épaisseurs équivalentes estimées lors de l'étape d).

L'étape h) peut comporter une caractérisation de différentes parties de l'objet respectivement associées à différents pixels. La caractérisation peut comporter :

- une formation d'une image montrant l'épaisseur équivalente régularisée d'un matériau de base ;
- et/ou une détermination d'un numéro atomique effectif à partir des épaisseurs équivalentes régularisées de chaque matériau de base ;
- et/ou un ratio entre une épaisseur équivalente régularisée d'un matériau de base sur la somme des épaisseurs équivalentes régularisées de chaque matériau de base.

Lors de l'étape b), le détecteur peut être est déplacé par rapport à l'objet ou l'objet peut être déplacé par rapport au détecteur. La source d'irradiation peut être déplacée par rapport à l'objet ou par rapport au détecteur.

Un deuxième objet de l'invention est un dispositif de caractérisation d'un objet comportant :

- une source d'irradiation, configurée pour émettre un rayonnement électromagnétique ionisant ;
- un support, destiné à recevoir un objet, de telle sorte que l'objet soit disposé entre la source d'irradiation et le détecteur ;
- un détecteur, comportant des pixels, le détecteur étant configuré pour détecter un rayonnement électromagnétique ionisant et à former, en plusieurs pixels, un spectre du rayonnement détecté ;
- un processeur, configuré pour recevoir les spectres formés par le détecteur et pour mettre en œuvre les étapes d) à h) d'un procédé selon le premier objet de l'invention.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

Les figures 1A et 1B représentent deux exemples de dispositif permettant une mise en œuvre de l'invention. La figure 1C montre un exemple d'objet de calibration pouvant être utilisé pour calibrer le procédé mise en œuvre dans l'invention.

La figure 2 montre les principales étapes d'un procédé selon l'invention.

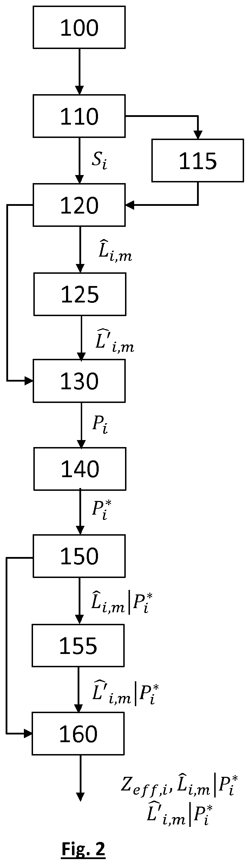

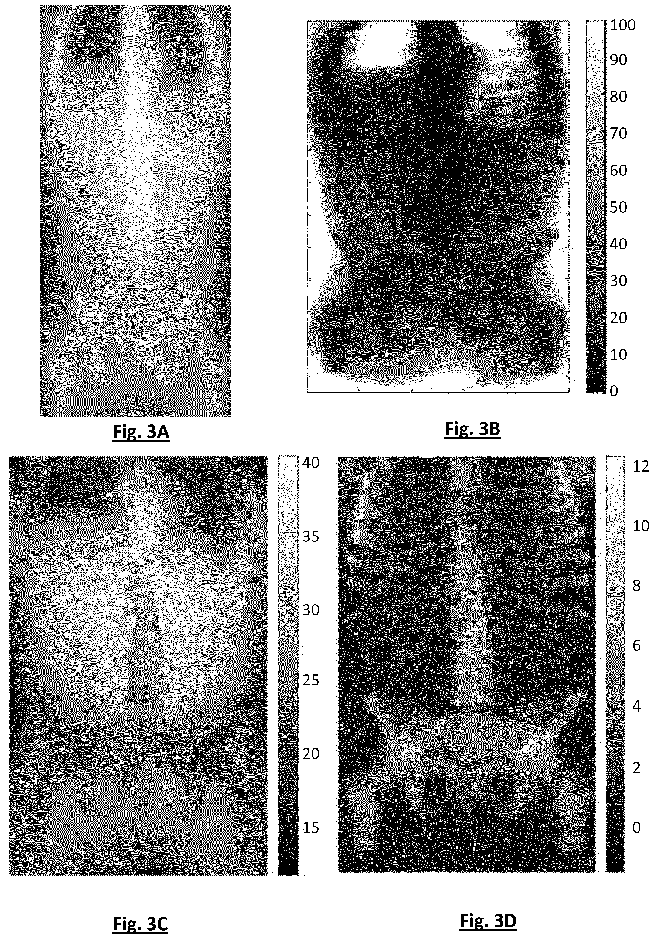

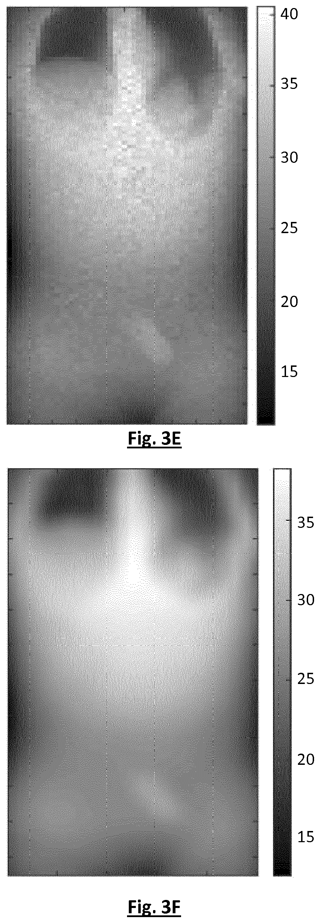

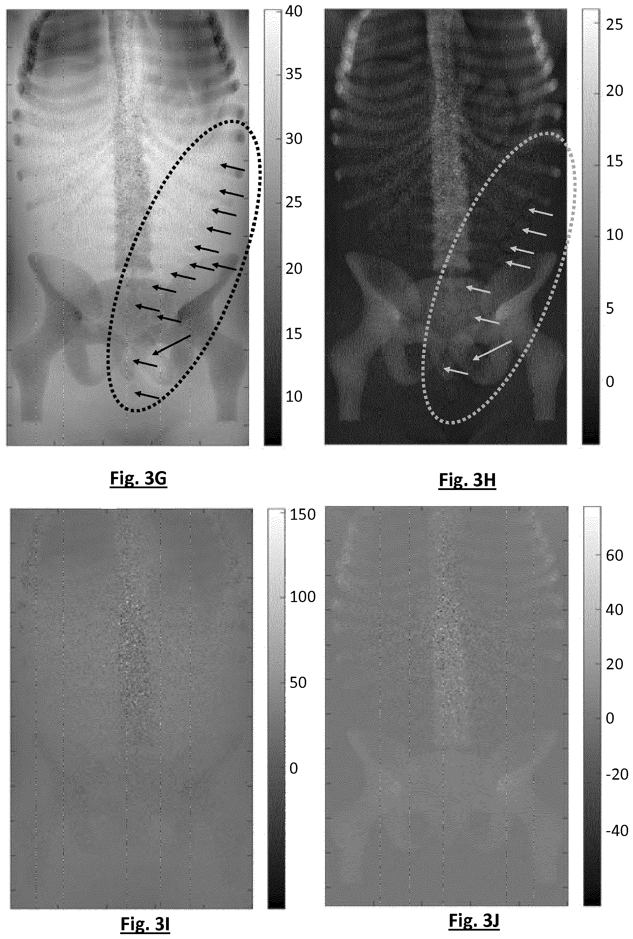

Les figures 3A à 3H sont des images simulées d'un fantôme, qui illustrent le déroulement du procédé décrit en lien avec la figure 2. La figure 3A est une radiographie du fantôme utilisé. La figure 3B montre une simulation du nombre de photons incidents aux pixels du détecteur au cours de l'essai. Les figures 3C et 3D montrent respectivement les distributions spatiales de l'épaisseur équivalente d'os et de l'épaisseur équivalente de tissu correspondant au fantôme représenté sur la figure 3A. La figure 3E représente une distribution spatiale d'un paramètre structurel obtenu à partir des épaisseurs équivalentes d'os et de tissu respectivement illustrées sur les figures 3C et 3D. Dans l'exemple représenté sur la figure 3E, le paramètre considéré est l'épaisseur de l'objet. La figure 3F montre un lissage spatial du paramètre évoqué en lien avec la figure 3E. Les figures 3G et 3H montrent respectivement les distributions spatiales de l'épaisseur équivalente d'os et de l'épaisseur équivalente de tissu du fantôme, ces épaisseurs équivalentes ayant été calculées en prenant en compte le paramètre lissé représenté sur la figure 3E.

Les figures 3I et 3J montrent respectivement les distributions spatiales de l'épaisseur équivalente d'os et de l'épaisseur équivalente de tissu du fantôme, ces épaisseurs équivalentes ayant été calculées selon un procédé de l'art antérieur.

Les figures 4A et 4B montrent une distribution spatiale du numéro atomique effectif du fantôme.

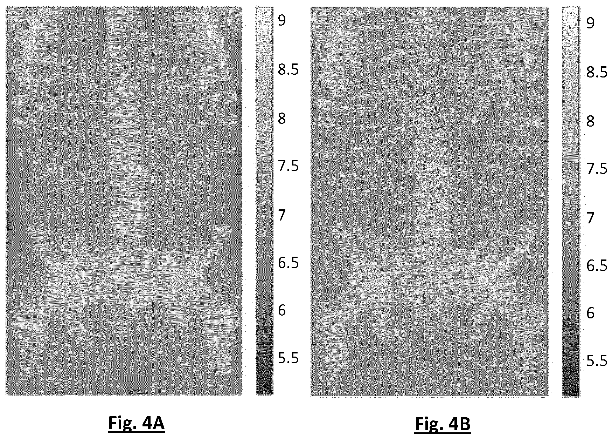

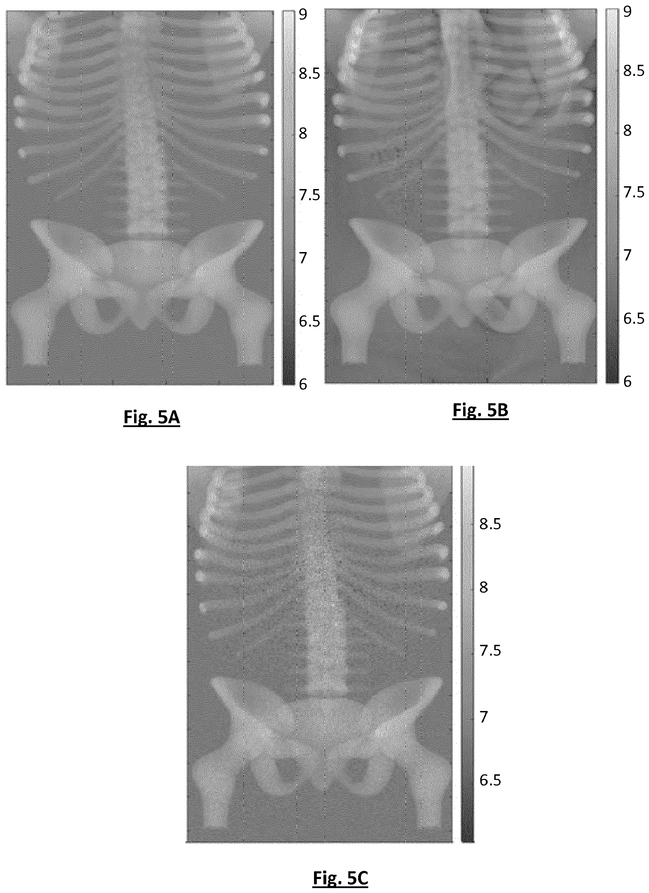

Les figures 4A et 4B ont été obtenues respectivement en mettant en œuvre et sans mettre en œuvre l'invention. Les figures 5A, 5B et 5C montrent une distribution spatiale d'un numéro atomique effectif du fantôme. Les figures 5A et 5C ont été obtenues sans mettre en œuvre l'invention, le nombre de photons incidents par pixel étant respectivement $10^6$ et $10^5$. La figure 5B a été obtenue en mettant en œuvre l'invention, le nombre de photons incidents par pixel étant de $10^5$.

## EXPOSE DE MODES DE REALISATION PARTICULIERS

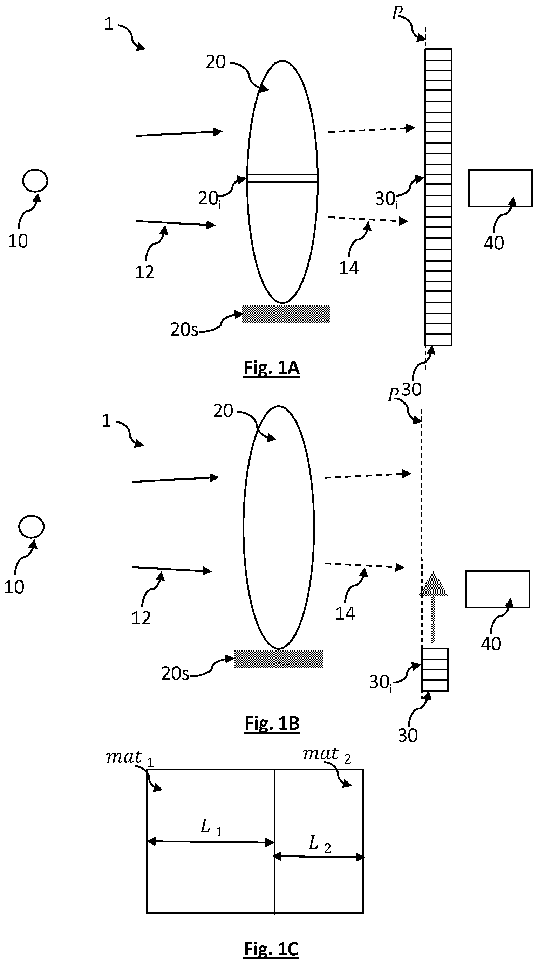

On a représenté, sur les figures 1A et 1B, des dispositifs 1 de caractérisation d'un objet 20 permettant une mise en œuvre de l'invention. Sur chacune de ces figures, le dispositif 1 comporte une source d'irradiation 10 et un détecteur 30. Il comporte également un support 20s, configuré pour recevoir un objet, de telle sorte que lorsque l'objet 20 est disposé sur le support 20s, il est interposé entre la source d'irradiation 10 et le détecteur 30.

La source d'irradiation 10 est configurée pour émettre un rayonnement électromagnétique ionisant 12, dit rayonnement incident, vers l'objet 20, ce dernier étant interposé entre la source d'irradiation 10 et le détecteur 30. Le détecteur 30 peut comporter des détecteurs élémentaires $30_i$ prenant la forme de pixels $30_i$ agencés selon un plan, dit plan de détection P. L'indice i désigne les coordonnées de chaque pixel dans le plan de détection. Les pixels peuvent s'étendre selon une ligne mais en général, ils s'étendent selon une matrice régulière bidimensionnelle.

L'objet 20 peut être un tissu biologique vivant, par exemple une partie du corps d'un animal ou d'un être humain. Le dispositif 1 est alors un dispositif d'imagerie médicale ou un portique de contrôle d'aéroport. L'objet peut également être une pièce industrielle ou un bagage, le dispositif étant alors utilisé à des fins de contrôle non destructif. Dans l'exemple qui suit, le dispositif 1 est un portique de contrôle d'aéroport, destiné à détecter une présence de substances illicites.

Le terme rayonnement électromagnétique ionisant désigne un rayonnement électromagnétique constitué de photons d'énergie supérieure à 1 keV, et de préférence inférieure à 5 MeV. La plage d'énergie du rayonnement ionisant peut être comprise entre 1 keV et 2 MeV, mais elle s'étend le plus souvent entre 1 keV et 150 keV ou 300 keV. Le rayonnement ionisant peut être un rayonnement X ou y. De préférence, la source d'irradiation 10 est polyénergétique, le rayonnement incident étant émis selon une plage d'énergie s'étendant généralement selon plusieurs dizaines voire centaines de keV. Il s'agit notamment d'un tube émetteur de rayons X.

L'objet 20 irradié par la source 10 transmet au détecteur 30 un rayonnement 14, dit rayonnement transmis, ou rayonnement atténué, ce dernier atteignant les pixels $30_i$. Chaque pixel $30_i$ est un détecteur spectrométrique élémentaire comportant :

- un matériau détecteur, apte à interagir avec les photons du rayonnement 14 transmis par l'objet, ce matériau étant de type scintillateur ou, de préférence, un matériau semiconducteur compatible avec une utilisation à la température ambiante, de type CdTe, CdZnTe ;
- un circuit électronique, apte à générer un signal dont l'amplitude dépend, et est de préférence proportionnelle, à l'énergie déposée par chaque photon interagissant dans le matériau détecteur ;
- un circuit de spectrométrie, apte à établir un spectre en énergie, noté $S_i$, des signaux détectés pendant une période temporelle, dite période d'acquisition.

Le dispositif de caractérisation 1 comporte également une unité de traitement 40 permettant la mise en œuvre des opérations de traitement décrites ci-après. L'unité de traitement 40 peut comprendre un microprocesseur et/ou des microcontrôleurs électroniques.

Sous l'effet de l'irradiation par le rayonnement incident 12, l'objet 20 transmet un rayonnement 14, dit rayonnement transmis, vers le détecteur 30. Chaque pixel $30_i$ forme un spectre en énergie $S_i$ du rayonnement transmis 14.

Le terme spectre en énergie $S_i$ correspond à un histogramme de l'amplitude des signaux détectés au cours de la période d'acquisition du spectre. Une relation entre l'amplitude A et l'énergie E peut être obtenue par une fonction d'étalonnage en énergie g telle que $E = g(A)$, selon des principes connus de l'homme du métier. Un spectre d'énergie $S_i$ est donc un vecteur, dont chaque terme $S_i(k)$ représente une quantité de rayonnement détecté par le pixel $30_i$ dans

une plage d'énergie $E_k \pm \dfrac{\partial E}{2},$ avec $dE$ étant la largeur spectrale de chaque canal $k$. $k$ désigne le rang du canal, avec $1 < k \leq K$, $K$ désignant le nombre de canaux du spectre.

Sur la figure 1B, on a représenté un détecteur 30 mobile par rapport à l'objet 20. Au cours d'une irradiation de l'objet 20, le détecteur est déplacé par rapport à l'objet 20, et acquiert des spectres du rayonnement 14 transmis par l'objet 20. Le déplacement du détecteur 30 est matérialisé par une flèche. Une telle configuration permet d'utiliser un détecteur avec un nombre réduit de pixels. Mais elle impose un déplacement du détecteur 30 par rapport à l'objet pour augmenter le champ d'observation. De façon alternative, l'objet 20 peut défiler entre la source de rayonnement 10 et le détecteur 30.

Ainsi, quel que soit le mode de réalisation, au cours de l'irradiation de l'objet 20, on acquiert des spectres en différents pixels $30_i$, chaque pixel $30_i$ correspondant à une partie $20_i$ de l'objet associée au pixel. On obtient ainsi une image spectrale de l'objet, dont chaque pixel est un spectre d'un rayonnement transmis par une partie $20_i$ de l'objet 20 associée au pixel $30_i$. Par partie de l'objet $20_i$ associée au pixel $30_i$, on entend une partie de l'objet vue par le pixel, c'est-à-dire située dans un champ d'observation du pixel, donc disposée dans un angle solide sous lequel le pixel $30_i$ voit l'objet 20. La partie de l'objet $20_i$ associée à un pixel $30_i$ est généralement située dans l'alignement entre la source d'irradiation 10 et le pixel $30_i$.

L'objet 20 peut être remplacé par un objet de calibration, composé d'un ou plusieurs matériaux de base, dont la nature et l'épaisseur sont connues. Un tel objet de calibration est représenté sur la figure 1C. Dans cet exemple, on a représenté un objet de calibration compotant une premier matériau de base *matl* d'épaisseur $L_1$ et un deuxième matériau de base *mat2* d'épaisseur $L_2$. Les matériaux de base sont des matériaux accessibles et aisément usinables, de façon à pouvoir disposer de différents objets de calibration, dans lesquels l'épaisseur de chaque matériau de base est variable. Les matériaux de base peuvent par exemple être du PVC (Polychlorure de Vinyle) ou du polyéthylène, ou du polypropylène. La calibration du procédé décrit ci-après consiste à disposer différents objets de calibration, de façon à obtenir des spectres de calibration $S_{cal}(L_1, L_2)$, en faisant varier $L_1$ et $L_2$. Chaque spectre de calibration $S_{cal}$ est un vecteur de dimension (K,1), où $K$ représente le nombre de canaux d'un spectre.

L'ensemble des spectres de calibration constitue une base de calibration. La base de calibration peut être complétée par des interpolations de façon à prendre en compte des épaisseurs de matériaux de base non disponibles sur les objets de calibration.

On va à présent décrire les principales étapes d'un procédé de caractérisation d'un objet 20, en lien avec la figure 2 et les figures 3A à 3H.

Etape 100 : irradiation de l'objet. L'objet 20 est irradié par la source d'irradiation 10.

Etape 110 : acquisition de spectres $S_i$ du rayonnement transmis par l'objet 30, par différents pixels $30_i$ du détecteur 30. Chaque pixel $30_i$ est associé à une partie $20_i$ de l'objet. Deux pixels différents sont respectivement associés à deux parties différentes de l'objet 20.

Etape 115 : regroupement de pixels. Cette étape est facultative. A chaque pixel $30_i$ est associé un groupe de pixels $G_j$. Le groupe de pixels $G_j$ auquel est associé le pixel $30_i$ est formé par des pixels adjacents du pixel $30_i$. Lorsque le détecteur est une matrice bidimensionnelle de pixels, on peut former $J$ groupes de pixels $G_j$. Chaque groupe de pixels comporte par exemple 5x5 pixels, ou 10x10 pixels. Les spectres $S_i$ acquis par les pixels d'un même groupe de pixels $G_j$ peuvent être additionnés, de façon à former un spectre $S_j$ représentatif du groupe de pixels. Cette opération correspond à une opération désignée par le terme "bining" connue de l'homme du métier. Cela permet de former un spectre $S_j$ présentant un meilleur rapport sur bruit que le spectre $S_i$ formé par chaque pixel élémentaire. En revanche, la résolution spatiale est dégradée. A l'issue de cette étape, le spectre $S_i$ associé à chaque pixel $30_i$ d'un même groupe de pixels $G_j$ est remplacé par le spectre $S_j$ établi pour groupe de pixels $G_j$.

Etape 120 : Décomposition de l'atténuation selon une base de matériaux.

Cette étape comporte une décomposition de l'atténuation $att_i$ selon une base de matériaux de calibration. Comme explicité en lien avec l'expression (1), cela revient à estimer, pour chaque pixel $30_i$, un couple d'épaisseurs équivalentes $(\hat{L}_{i,1}, \hat{L}_{i,2})$, de telle sorte que :

$$att_i \approx \hat{L}_{i,1}\mu_1 + \hat{L}_{i,2}\mu_2 \ (2)$$

Plus généralement, lorsque les spectres $S_i$ sont définis selon $K$ canaux, $K$ étant un entier supérieur ou égal à 2, l'atténuation peut être décomposée selon un nombre $M$ d'épaisseurs, $\hat{L}_{i,m=1}...\hat{L}_{i,m=M}$, dites épaisseurs équivalentes, de $M$ matériaux de calibration différents, avec $M \leq K$. $M$ désigne le nombre de matériaux de calibration considérés. Le procédé comporte alors une estimation des $M$ épaisseurs équivalentes $\hat{L}_{i,m}$ avec :

$$att_i \approx \sum_{m=1}^{M} \hat{L}_{i,m}\mu_m \ (3)$$

où $\mu_m$ est une fonction spectrale d'atténuation linéique du matériau de calibration $mat_m$.

Lorsqu'un spectre $S_i$ est acquis selon des paramètres d'acquisition identiques à chaque spectre de calibration $S_{cal}$, les épaisseurs équivalentes correspondant au spectre acquis $S_i$ peuvent être obtenues en identifiant le spectre de la base de calibration le plus proche du spectre acquis. Par paramètres d'acquisition identiques, on entend : même source d'irradiation, même détecteur, même durée d'acquisition, mêmes distances entre la source d'irradiation et l'objet et entre l'objet et le détecteur.

Les épaisseurs équivalentes peuvent être estimées selon le procédé décrit dans le document EP3106864, et plus précisément entre les paragraphes [0060] à [0081] de ce document. Si $S_{cal}(L_1 \ldots L_M)$ désigne un spectre de la base de calibration, obtenu en utilisant un objet de calibration comportant respectivement des épaisseurs $L_1 \ldots L_M$ de matériaux de base $mat_1 \ldots mat_M$, on peut définir, pour chaque spectre $S_i$, une fonction de vraisemblance $V_i$ telle que :

$$ln\left(V_i\left(S_i, \, S_{cal}(L_1 \ldots L_M)\right)\right) = \left(-\sum_{k=1}^{K} S_{cal}(L_1 \ldots L_M) + \sum_{k=1}^{K}(S_i \times ln\,(S_{cal}(L_1 \ldots L_M)))\right) (4).$$

Les épaisseurs équivalentes $\hat{L}_{i,m=1} \ldots \hat{L}_{i,m} \ldots \hat{L}_{i,m=M}$ correspondant au spectre $S_i$ mesuré par chaque pixel $30_i$ sont celles maximisant la fonction de vraisemblance. Ainsi,

$$\hat{L}_{i,m=1} \ldots \hat{L}_{i,m=M} = argmax\left(ln\left(V_i\left(S_i, \, S_{cal}(L_1 \ldots L_M)\right)\right)\right) (5).$$

et

$$-ln\left(\frac{S_i}{S_o}\right) = att_i \approx \sum_{m=1}^{M} \hat{L}_{i,m}\mu_m \,(6)$$

$S_o$ étant un spectre du rayonnement détecté par le détecteur 30 en l'absence d'objet disposé entre la source d'irradiation 10 et le détecteur 30.

Selon l'expression (6), l'atténuation $att_i$ correspondant au spectre $Sp_i$ mesuré par chaque pixel $30_i$ peut être décomposée dans une base de matériaux de calibration $mat_1 \ldots mat_M$, l'atténuation pouvant être assimilée à une somme des atténuations linéiques de chaque matériau de la base de calibration, pondérée par les épaisseurs équivalentes respectivement associées à chaque matériau de base.

Etape 125 Changement de base.

Les épaisseurs équivalentes établies lors de l'étape 120 sont respectivement associées à des matériaux de base $mat_1 \ldots mat_M$ utilisés lors de la calibration, c'est-à-dire des matériaux de calibration. Les matériaux de base utilisés au cours de la calibration forment une base de départ. On peut procéder à un changement de base, de façon que le spectre soit exprimé en fonction de matériaux de base susceptibles de composer l'objet. Par exemple, lorsque l'objet analysé est un corps d'un animal ou d'un d'individu, les matériaux de base peuvent être des matériaux $mat'_1 \ldots mat'_M$ représentatifs d'éléments physiologiques, par exemple l'os ou des tissus. Les épaisseurs équivalentes $\hat{L}_{i,m=1} \ldots \hat{L}_{i,m} \ldots \hat{L}_{i,m=M}$ exprimées dans la base de départ $mat_1 \ldots mat_m \ldots mat_M$ peuvent être exprimées dans une base d'arrivée $mat'_1 \ldots mat'_m \ldots mat'_M$. Le changement de base est obtenu par :

$$\begin{bmatrix} \hat{L}'_{i,m=1} \\ \vdots \\ \hat{L}'_{i,m=M} \end{bmatrix} = T \begin{bmatrix} \hat{L}_{i,m=1} \\ \vdots \\ \hat{L}_{i,m=M} \end{bmatrix} (7)$$

Où $\hat{L}'_{i,m=1} \ldots \hat{L}'_{i,m} \ldots \hat{L}'_{i,m=M}$ sont les épaisseurs équivalentes dans la base d'arrivée $mat'_1 \ldots mat'_m \ldots mat'_M$ et $T$ est une matrice de changement de base, de dimension $(M,M)$.

La matrice T peut être obtenue en connaissant les fonctions spectrales d'atténuation linéique de chaque matériau de base. Soit Y, une matrice des fonctions spectrales d'atténuation linéique des matériaux de la base de départ $mat_1 \ldots mat_M$, et Z une matrice des fonctions spectrales d'atténuation linéique des matériaux de la base d'arrivée $mat'_1 \ldots mat'_M$.

$$Y = \begin{bmatrix} \mu_{mat_1}(E_1) & \cdots & \mu_{mat_M}(E_1) \\ \vdots & \ddots & \vdots \\ \mu_{mat_1}(E_K) & \cdots & \mu_{mat_M}(E_K) \end{bmatrix} \text{et } Z = \begin{bmatrix} \mu_{mat'_1}(E_1) & \cdots & \mu_{mat'_M}(E_1) \\ \vdots & \ddots & \vdots \\ \mu_{mat'_1}(E_K) & \cdots & \mu_{mat'_M}(E_K) \end{bmatrix}$$

$$Y = Z.T \,(8)$$

Y et Z sont des matrices de dimension $(K, M)$ et T est une matrice de changement de base de dimension $(M, M)$ et . désigne le produit matriciel.

La matrice de changement de base T peut être déterminée par une méthode de type moindre carrés, de telle sorte que :

$$T = Z^*.Y \ (9)$$

où Z* est la pseudo inverse de Z : $Z^* = (Z^t.Z)^{-1}.Z^t$ (10) où t désigne l'opérateur transposée.

A partir d'une base de départ, composée de matériaux de type polypropylène et PVC, il est possible d'exprimer les épaisseurs équivalentes dans une base d'arrivée composée de tissus mous et d'os, en mettant en œuvre l'équation (8) et en utilisant une matrice de changement de base T telle que :

$$T = \begin{pmatrix} 1.11 & -0.97 \\ -0.09 & 1.55 \end{pmatrix}$$

L'étape 125 est optionnelle. Les étapes suivantes peuvent être mises en œuvre à partir des épaisseurs équivalentes dans la base de départ, c'est-à-dire dans la base formée par les matériaux de calibration, ou dans la base d'arrivée, comportant des matériaux différents des matériaux de calibration, davantage représentatifs de l'objet observé.

On a simulé une mise en œuvre des étapes 100 à 125, à l'aide d'un fantôme simulant un thorax et un abdomen d'un individu, dans lequel sont insérées des capsules de drogue. Les capsules simulées comportent de la cocaïne pure ($C_{17}H_{21}NO_4$) mélangée à un adultérant ($C_{11}H_{12}N_2S$). Les fractions massiques de cocaïne pure et d'adultérant sont de 50% et 50%. L'application visée est la détection de la présence de drogue portée par un individu, par un contrôle de type portique d'aéroport. La figure 3A montre une radiographie simulée du fantôme. On a simulé l'irradiation du fantôme par une source d'irradiation de type générateur de rayon X porté à un potentiel de 160 KV. La source d'irradiation 10 est couplée à un filtre d'aluminium d'épaisseur 2mm, de façon à filtrer les photons de faible énergie. En l'absence d'objet 20, le nombre de photons atteignant chaque pixel est égal à 6000. On a simulé les spectres acquis par un capteur de type CdTe d'épaisseur 3 mm, formant une matrice de 500 x 1152 pixels, la surface exposée de chaque pixel étant de 0.8 x 0.8 mm. Chaque spectre comporte 64 canaux en énergie. La figure 3B montre le nombre moyen de photons reçus au niveau de chaque pixel.

On a simulé, pour chaque pixel $30_i$, le spectre $S_i$ du rayonnement 14 atténué par l'objet. Le spectre $S_i$ est représentatif de l'atténuation $att_i$ d'une partie $20_i$ de l'objet associée à chaque pixel. Chaque pixel $30_i$ a été regroupé, de façon à former des groupes de 10x10 pixels. On a remplacé le spectre $S_i$ de chaque pixel par une addition des spectres du groupe de pixels comportant ledit pixel. On a ensuite décomposé l'atténuation $att_i$ correspondant à chaque spectre $S_i$ en utilisant une base de matériaux de calibration formée de PVC et de polypropylène, de façon à estimer des épaisseurs équivalentes $\hat{L}_{i,1}, \hat{L}_{i,2}$ pour chaque pixel $30_i$. On a ensuite procédé à un changement de base, de façon à obtenir des épaisseurs équivalentes $\hat{L}'_{i,1}, \hat{L}'_{i,2}$ de tissu et d'os. Les figures 3C et 3D représentent respectivement les épaisseurs équivalentes $\hat{L}'_{i,1}, \hat{L}'_{i,2}$ de tissu et d'os obtenues à partir de spectres. Sur ces figures, les niveaux de gris correspondent à des épaisseurs exprimées en cm.

Etape 130. Calcul d'un paramètre structurel de l'objet.

Au cours de cette étape, les épaisseurs équivalentes résultant de l'étape 120 ou de l'étape 125 sont combinées, en chaque pixel, de manière à calculer, en chaque pixel $30_i$, un paramètre structurel $P_i$ de l'objet. Plus précisément, il s'agit de combiner les épaisseurs $\hat{L}_{i,1} \dots \hat{L}_{i,m} \dots \hat{L}_{i,M}, \hat{L}'_{i,1} \dots \hat{L}'_{i,m} \dots \hat{L}'_{i,M}$ déterminées en chaque pixel $30_i$, pour calculer un paramètre structurel de la partie de l'objet $20_i$ associée au pixel.

Le paramètre structurel $P_i$ peut être une dimension de l'objet, par exemple une épaisseur, ou une composition de l'objet. Le paramètre structurel $P_i$ est une fonction f des épaisseurs préalablement déterminées. Ainsi,

$$P_i = f\left(\hat{L}_{i,1} \dots \hat{L}_{i,M}\right) \ (11)$$

ou

$$P_i = f\left(\hat{L}'_{i,1} \dots \hat{L}'_{i,M}\right) \ (11').$$

Selon un premier exemple, le paramètre structurel est une épaisseur de l'objet. Selon cet exemple, le paramètre $P_i$ calculé, en chaque pixel $30_i$, est une somme des épaisseurs résultant de l'étape 120 ou 125. Par exemple,

$$P_i = \sum_{m=1}^{m=M} \hat{L}_{i,m} \ (12)$$

où

$$P_i = \sum_{m=1}^{m=M} \widehat{L}'_{i,m} \ (12').$$

Selon cet exemple, le paramètre $P_i$ est l'épaisseur de la partie de l'objet $20_i$ associée au pixel $30_i$. La figure 3E représente une réalisation de cet exemple, à partir des épaisseurs de tissu $\widehat{L}'_{i,1}$ et d'os $\widehat{L}'_{i,2}$ obtenues, pour chaque pixel $30_i$. Sur la figure 3E, le niveau de gris correspond à la valeur du paramètre structurel en chaque pixel, c'est-à-dire l'épaisseur de l'objet calculée au niveau de chaque pixel. Cette dernière correspond à l'épaisseur de la partie de l'objet $20_i$ associée à chaque pixel $30_i$.

A l'issue de l'étape 130, on dispose d'un maillage spatial du paramètre structurel $P_i$, ce dernier étant calculé pour chaque pixel ou pour chaque groupe de pixels.

Etape 140 : lissage spatial du paramètre structurel de l'objet

Au cours de cette étape, le paramètre structurel de l'objet $P_i$, déterminé sur chaque pixel au cours de l'étape 130, subit un lissage spatial. L'idée sous-jacente est que la structure de l'objet examiné ne subit pas de variation brusque, et que deux paramètres déterminés respectivement pour deux pixels adjacents, c'est-à-dire pour deux parties adjacentes de l'objet, ne peuvent pas connaître une évolution discontinue. Une telle hypothèse est particulièrement pertinente lorsque le paramètre structurel est l'épaisseur de l'objet, l'objet étant une portion d'un animal ou d'un individu. L'étape 140 est un lissage spatial du maillage du paramètre structurel $P_i$ résultant de l'étape 130, de manière à obtenir, pour chaque pixel, un paramètre structurel lissé $P_i^*$. Un tel lissage spatial peut être effectué en mettant en œuvre des filtres de lissage connus de l'homme du métier, par exemple un filtre gaussien, un filtre médian ou un filtre de type Savitzky-Golay. La figure 3F montre un lissage spatial de type Savitzky Golay opéré sur le maillage spatial du paramètre structurel $P_i$ représenté sur la figure 3E. Dans cet exemple, le filtre de Savitzky Golay était réalisé selon une fenêtre de 11 pixels en utilisant un polynôme de degré 7. Sur la figure 3F, le niveau de gris correspond à la valeur du paramètre lissé $P_i^*$ en chaque pixel, c'est-à-dire l'épaisseur de l'objet, au niveau de chaque pixel, après lissage spatial. Cette étape peut comporter une interpolation spatiale, en particulier lorsque le paramètre structurel n'est établi que sur certains pixels distants les uns des autres.

A l'issue de l'étape 140, on dispose d'un maillage spatial d'un paramètre structurel lissé $P_i^*$, ce dernier étant calculé pour chaque pixel $30_i$, c'est-à-dire pour chaque partie $20_i$ de l'objet respectivement associée à un pixel.

Etape 150 : deuxième décomposition selon une base de matériaux.

Cette étape est similaire à l'étape 120. Cependant, alors que l'étape 120 est mise en œuvre sans a priori, l'étape 150 est réalisée en utilisant la valeur du paramètre structurel lissé $P_i^*$ déterminé en chaque pixel. Au cours de l'étape 150, on détermine les épaisseurs équivalentes $\widehat{L}_{i,m=1}\big|P_i^* \ ... \ \widehat{L}_{i,m}\big|P_i^* \ ... \ \widehat{L}_{i,m=M}\big|P_i^*$ correspondant au spectre $S_i$, comme décrit en lien avec l'étape 120. Cependant, et cela constitue un élément important de l'invention, on prend en compte la valeur, pour chaque pixel $30_i$, du paramètre structurel lissé $P_i^*$, cette dernière reliant les épaisseurs équivalentes $\widehat{L}_{i,m=1}\big|P_i^* \ ... \ \widehat{L}_{i,m}\big|P_i^* \ ... \ \widehat{L}_{i,m=M}\big|P_i^*$ correspondant au pixel considéré. La notation $\widehat{L}_{i,m}\big|P_i^*$ désigne une épaisseur équivalente $\widehat{L}_{i,m}$ sachant le paramètre structurel lissé $P_i^*$. Lorsque le paramètre structurel considéré est l'épaisseur de l'objet, les épaisseurs équivalentes $\widehat{L}_{i,m=1}\big|P_i^* \ ... \ \widehat{L}_{i,m}\big|P_i^* \ ... \ \widehat{L}_{i,m=M}\big|P_i^*$ sont estimées sachant que $\sum_{m=1}^{m=M} \widehat{L}_{i,m} = P_i^*$. Il s'agit donc d'une estimation sous contrainte, les grandeurs estimées étant telles que $f(\widehat{L}_{i,1} \ ... \ \widehat{L}_{i,M}) = P_i^*$.

Lorsque les épaisseurs équivalentes sont estimées en maximisant une fonction de vraisemblance, l'expression (5) est remplacée par :

$$\hat{L}_{i,m=1}\big|P_i^*...\hat{L}_{i,m=M}\big|P_i^* = argmax\left(ln\left(V_i\big(S_i,\ S_{cal}(L_1\ ...\ L_M)\big)\right) - \lambda g\big(f(L_1\ ...\ L_M), P_i^*\big)\right) \quad (15)$$

où :

- $g\big(f(L_1\ ...\ L_M), P_i^*\big)$ est une fonction de régularisation, prenant en compte la fonction *f* décrite dans l'expression (11) et la valeur du paramètre lissé $P_i^*$ au pixel considéré 30$_i$. Selon un premier exemple, $g\big(L_1\ ...\ L_M, P_i^*\big)$ impose $P_i^* = f(L_1\ ...\ L_M).$ Selon un autre exemple, la contrainte exercée par la fonction de régularisation *g* est plus faible. La fonction de régularisation *g* peut être une loi de probabilité appliquée à $f(L_1\ ...L_M)$, et dont la valeur moyenne est $P_i^*.$ Par exemple, la fonction *g* impose à $f(L_1\ ...\ L_M)$ de suivre une loi gaussienne centrée sur $P_i^*$, avec un écart type prédéfini.

- $\lambda$ est un facteur de pondération pondérant la prise en compte de la fonction de régularisation *g* ;

- $\hat{L}_{i,m=1}\big|P_i^*...\hat{L}_{i,m=M}\big|P_i^*$ sont des épaisseurs équivalentes régularisées sachant le paramètre lissé $P_i^*.$

A l'issue de l'étape 150, on dispose, pour chaque pixel 30$_i$ d'épaisseurs $\hat{L}_{i,m=1}\big|P_i^*...\hat{L}_{i,m=M}\big|P_i^*$ dites régularisées par le paramètre lissé $P_i^*.$ Les épaisseurs régularisées sont calculées pour chaque pixel. Cependant, à la différence de l'art antérieur, par la prise en compte du paramètre lissé $P_i^*$, les épaisseurs régularisées tiennent compte de la structure de l'objet.

Etape 155 : changement de base. A partir des épaisseurs équivalentes $\hat{L}_{i,m=1}\big|P_i^*...\hat{L}_{i,m=M}\big|P_i^*$ dans la base des matériaux de calibration, on peut obtenir des épaisseurs équivalentes $\widehat{L'}_{i,m=1}\big|P_i^*...\widehat{L'}_{i,m=M}\big|P_i^*$ dans une autre base, en appliquant l'expression (8) décrite en lien avec l'étape 125. Cette étape est facultative.

Les figures 3G et 3H représentent respectivement les épaisseurs équivalentes $\widehat{L'}_{i,m=1}\big|P_i^*...\widehat{L'}_{i,m=M}\big|P_i^*$ de tissu et d'os obtenues, pour chaque pixel 30$_i$ en prenant en compte le paramètre lissé $P_i^*.$ Sur ces figures, les niveaux de gris correspondent à des épaisseurs exprimées en cm.

Etape 160 : caractérisation de l'objet.

A partir des épaisseurs équivalentes régularisées issues de l'étape 150 ou de l'étape 155, on peut caractériser l'objet 20. La caractérisation peut être effectuée directement à partir des épaisseurs équivalentes $\hat{L}_{i,m=1}\big|P_i^*...\hat{L}_{i,m=M}\big|P_i^*$ ou $\widehat{L'}_{i,m=1}\big|P_i^*...\widehat{L'}_{i,m=M}\big|P_i^*.$

Dans l'exemple illustré, la caractérisation de l'objet peut être établie à partir des figures 3G et 3H. Plus précisément, sur ces figures, on remarque la présence de formes suspectes, correspondant aux capsules disposées dans le fantôme. Ces formes sont situées au niveau de l'abdomen, dans une zone contournée par une ligne en pointillés. On a repéré certaines d'entre elles par une flèche.

Les figures 3I et 3J sont représentatives de l'art antérieur, et en particulier d'un procédé tel que décrit dans EP3106864. Les figures 3G et 3H correspondent à une mise en œuvre d'invention. Les comparaisons entre les figures 3G et 3I d'une part ainsi que 3H et 3J d'autre part, montrent le gain en qualité d'image apporté par l'invention. L'amélioration apportée est indéniable. Le procédé décrit ci-avant permet une localisation de capsules tout en exposant l'objet à une exposition faible, de l'ordre de 1 $\mu$Gy.

Ainsi, il est possible de caractériser l'objet 20 directement à partir d'une image représentant les épaisseurs équivalentes régularisées, obtenues lors des étapes 150 ou 155.

A partir des épaisseurs équivalentes régularisées, il est possible d'estimer un numéro atomique effectif des différentes parties 20$_i$ de l'objet 20. Le numéro atomique effectif $Z_{eff}$ a par exemple été décrit dans le document EP3084406. Il s'applique à un composé chimique, et correspond une combinaison des numéros atomiques des corps simples constitutifs du composé, chaque numéro atomique étant affecté d'un coefficient de pondération dépendant de la fraction massique ou atomique du corps simple dans le composé. Il est possible d'estimer le numéro atomique effectif à partir des épaisseurs équivalentes régularisées, issues des étapes 150 ou 155, selon l'expression :

$$Z_{eff,i} = \left( \frac{\sum_{m=1}^{M} \rho_m \hat{L}_{i,m} |P_i^*| Z_{effm}^p}{\sum_{m=1}^{M} \rho_m \hat{L}_{i,m} |P_i^*|} \right)^{1/p} \quad (16)$$

où :

- $p = 3$;
- $Z_{effm}$ est le numéro atomique effectif de chaque matériau de base $mat_m$ ;
- $\rho_m$ est la densité du matériau de base $mat_m$.

Ou :

$$Z_{eff,i} = \left( \frac{\sum_{m=1}^{M} \rho'_m \hat{L'}_{i,m} |P_i^*| {Z'}_{effm}^p}{\sum_{m=1}^{M} \rho'_m \hat{L'}_{i,m} |P_i^*|} \right)^{1/p} \quad (16')$$

où :

- $p = 3$;
- $Z'_{effm}$ est le numéro atomique effectif de chaque matériau de base $mat'_m$ ;
- $\rho'_m$ est la densité du matériau de base $mat'_m$.

Les figures 4A et 4B sont des images représentant des estimations du numéro atomique effectif du fantôme précédemment décrit. Sur les figures 4A et 4B, les estimations sont effectuées en mettant en œuvre l'expression (16') à partir d'épaisseurs équivalentes respectivement issues de l'étape 155, en mettant en œuvre l'invention, sans l'étape 115, et selon l'art antérieur. On observe que la figure 4A permet d'identifier certaines capsules. Par ailleurs, l'estimation du numéro atomique effectif à partir d'épaisseurs équivalentes régularisées, obtenues en mettant en œuvre l'invention, est moins bruitée que l'estimation effectuée à partir d'épaisseurs équivalentes obtenues selon l'art antérieur.

Les figures 5A à 5C sont des images représentant des estimations du numéro atomique effectif du fantôme, sans capsule, en faisant varier le niveau d'irradiation auquel est exposé le fantôme. La figure 5A a été obtenue en utilisant des épaisseurs équivalentes estimées selon l'art antérieur, le nombre de photons incidents par pixels étant de $10^6$. La figure 5B a été obtenue en utilisant des épaisseurs équivalentes régularisées estimées en mettant en œuvre l'invention, le nombre de photons incidents par pixels étant de $10^5$. La figure 5C a été obtenue en utilisant des épaisseurs équivalentes estimées selon l'art antérieur, le nombre de photons incidents par pixels étant de $10^5$.

A partir des figures 5A et 5B, on constate que l'invention permet d'obtenir une image de qualité équivalente à l'art antérieur, tout en réduisant l'exposition d'un facteur 10. Cela est particulièrement important lorsque l'objet analysé est une portion d'un animal ou un individu vivant, car cela permet d'obtenir des images exploitables tout en réduisant l'exposition au rayonnement ionisant.

A partir des figures 5B et 5C, on constate que l'invention permet d'obtenir, à niveau d'irradiation constant, une image de qualité supérieure à l'art antérieur.

Dans les exemples qui précèdent, le paramètre structurel $P_i$ déterminé lors de l'étape 130 et lissé lors de l'étape 140 est l'épaisseur de l'objet analysé, ou plus précisément l'épaisseur des parties $20_i$ de l'objet respectivement associées à chaque pixel $30_i$.

Selon un autre exemple, le paramètre structurel $P_i$ concerne la composition de l'objet. Il peut s'agir du numéro atomique effectif $Z_{eff,i}$, ce dernier étant obtenu à partir des épaisseurs équivalentes régularisées selon l'expression (16) ou (16'). Il peut également s'agir d'une proportion relative d'un matériau de base relativement à l'ensemble des matériaux de base. Par exemple, le paramètre $P_i$ peut être un ratio de l'épaisseur équivalente régularisée $\hat{L}_{1,i}$ correspondant au premier matériau de base, sur une somme des épaisseurs équivalentes régularisées correspondant à l'ensemble des matériaux de base $mat_m$. Ainsi,

$$P_i = \frac{\hat{L}_{1,i}}{\sum_{m=1}^{M} \hat{L}_{m,i}} \quad (17)$$

Un tel paramètre peut par exemple être utilisé pour effectuer un tri rapide d'objets en fonction de leur composition. Une application peut être un tri de déchets, en fonction de la présence ou non d'additifs, ou un tri parmi des pièces métalliques. Par exemple, les pièces métalliques peuvent être des alliages d'aluminium, que l'on souhaite trier en fonction de la présence d'éléments d'alliages particulier, par exemple en fonction de la présence de cuivre ou de zinc. Dans ces applications, la présence d'éléments d'alliages particuliers, ou d'additifs, entraîne une variation du paramètre structurel, tel que défini par les expressions (16), (16') ou (17).

L'invention permet d'effectuer un tri nécessitant une irradiation faible, ce qui limite la durée du contrôle et permet d'augmenter la cadence selon laquelle est effectué le tri.

Quelle que soit l'application visée, l'invention permet une caractérisation d'un objet en nécessitant une exposition réduite de ce dernier par rapport à l'art antérieur. L'invention pourra être mise en œuvre dans des applications médicales, par exemple à des fins d'aide au diagnostic, ou dans des applications liées au contrôle non destructif.

## Revendications

1. Procédé de caractérisation d'un objet (20) comportant les étapes suivantes :

   a) disposition de l'objet entre une source d'irradiation (10) et un détecteur de rayonnement (30), la source d'irradiation étant configurée pour émettre un rayonnement électromagnétique ionisant (12), de type X ou gamma, se propageant jusqu'à l'objet ;
   b) irradiation de l'objet par la source d'irradiation (10) et détection d'un rayonnement transmis par l'objet (14) à l'aide du détecteur de rayonnement, le détecteur de rayonnement comportant plusieurs pixels (30$_i$), chaque pixel (30$_i$) étant associé à une partie (20$_i$) de l'objet ;
   c) pour chaque pixel (30$_i$), formation d'un spectre d'énergie ($S_i$) du rayonnement détecté, chaque spectre comportant au moins deux bandes d'énergie distinctes ;
   d) à partir de chaque spectre formé lors de l'étape c), estimation, en chaque pixel, d'au moins deux épaisseurs équivalentes ($\hat{L}_{i},1 ... \hat{L}_{i,M}, \hat{L}'_{i,1} ... \hat{L}'_{i,M}$) respectivement associées à au moins deux matériaux de base ($mat_1 ... mat_M, mat'_1 ... mat'_M$);

   le procédé étant **caractérisé en ce qu'**il comporte, suite à l'étape d), les étapes suivantes :

   e) à partir des épaisseurs équivalentes résultant de l'étape d), calcul d'un paramètre structurel de l'objet ($P_i$) en différents pixels (30$_i$), le paramètre structurel de l'objet étant pour chaque pixel :

      - une épaisseur ($L_i$) de la partie de l'objet (20$_i$) associée au pixel ;
      - ou représentatif d'une composition de la partie de l'objet (20$_i$) associée au pixel.

   f) lissage spatial du paramètre structurel calculé en plusieurs pixels, de façon à associer, à chaque pixel, un paramètre structurel lissé $(P_i^*)$;
   g) à partir du paramètre structurel lissé en chaque pixel, et à partir de chaque spectre formé lors de l'étape c), estimation, en chaque pixel, d'épaisseurs équivalentes régularisées $(\hat{L}_{i,m=1}|P_i^*...\hat{L}_{i,m=M}|P_i^*, \hat{L}'_{i,m=1}|P_i^*...\hat{L}'_{i,m=M}|P_i^*)$ respectivement associées à chaque matériau de base ;
   h) caractérisation de l'objet (20) à partir des épaisseurs équivalentes régularisées estimées lors de l'étape g).

2. Procédé selon la revendication 1, dans l'étape d) et/ou l'étape g) comporte, pour chaque pixel (30$_i$), une prise en compte de spectres de calibration ($S_{cal}(L_1, L_2)$), chaque spectre de calibration étant associé à une épaisseur de chaque matériau de base.

3. Procédé selon la revendication 2, dans lequel l'étape d) et/ou l'étape g) comporte également, pour chaque pixel (30$_i$):

   - un calcul d'une fonction de vraisemblance ($V_i(S_i, S_{cal}(L_1, L_2))$) à partir du spectre ($S_i$) formé par le pixel lors de l'étape c) et à partir des spectres de calibration ($S_{cal}(L_1, L_2)$); chaque spectre de calibration étant associé à au moins un matériau de calibration ($mat_1 ... mat_m ... mat_M$) d'une épaisseur connue ;
   - une détermination d'une épaisseur équivalente de chaque matériau de calibration maximisant la fonction de vraisemblance, chaque matériau de calibration formant un matériau de base.

**4.** Procédé selon la revendication 3, dans lequel l'étape d) et/ou l'étape g) comporte un changement de base, entre une base de départ, formé par les matériaux de calibration ($mat_1 \dots mat_m \dots mat_M$), et une base d'arrivée $emat'_1 \dots mat'_m \dots mat'_M$), formée par des matériaux de base représentatifs de l'objet, de façon à obtenir une épaisseur équivalente ($\hat{L}'_{i,1} \dots \hat{L}'_{i,M}$) de chaque matériau de la base d'arrivée .

**5.** Procédé selon l'une quelconque des revendications précédentes, dans lequel l'étape d) comporte :

- un regroupement de pixels adjacents, pour former un groupe de pixels ($G_j$);
- une association, à chaque groupe de pixels, d'un spectre regroupé ($S_j$), combinant les spectres formés pour chaque pixel du groupe de pixels ;

de telle sorte que l'étape d) est mise en œuvre, pour au moins un pixel d'un groupe de pixels, à partir du spectre regroupé associé au groupe de pixels.

**6.** Procédé selon l'une quelconque des revendications précédentes, dans lequel le paramètre structurel ($P_i$) représente une composition de la partie ($20_i$) de l'objet associée au pixel, et dans lequel le paramètre structurel, déterminé en chaque pixel :

- est un numéro atomique effectif ($Z_{eff,i}$), déterminé à partir des épaisseurs équivalentes estimées lors de l'étape d) ;
- ou comporte un ratio entre une épaisseur équivalente d'un matériau de base sur la somme des épaisseurs équivalentes estimées lors de l'étape d).

**7.** Procédé selon l'une quelconque des revendications précédentes, dans lequel l'étape h) comporte une caractérisation des différentes parties ($20_i$) de l'objet respectivement associées à différents pixels.

**8.** Procédé selon la revendication 7, dans lequel la caractérisation comporte :

- une formation d'une image montrant l'épaisseur équivalente régularisée d'un matériau de base ;
- et/ou une détermination d'un numéro atomique effectif ($Z_{eff,i}$) à partir des épaisseurs équivalentes régularisées de chaque matériau de base ;
- et/ou un ratio entre une épaisseur équivalente régularisée d'un matériau de base sur la somme des épaisseurs équivalentes régularisées de chaque matériau de base.

**9.** Procédé selon l'une quelconque des revendications précédentes dans lequel lors de l'étape b), le détecteur est déplacé par rapport à l'objet ou l'objet est déplacé par rapport au détecteur.

**10.** Dispositif de caractérisation d'un objet (20) comportant :

- une source d'irradiation (10), configurée pour émettre un rayonnement électromagnétique ionisant ;
- un support (20s), destiné à recevoir l'objet, de telle sorte que l'objet soit disposé entre la source d'irradiation et le détecteur ;
- un détecteur (30), comportant des pixels, le détecteur étant configuré pour détecter un rayonnement électromagnétique ionisant et à former, en plusieurs pixels ($30_i$), un spectre ($S_i$) du rayonnement détecté ;
- un processeur (40), configuré pour recevoir les spectres formés par le détecteur et pour mettre en œuvre les étapes d) à h) d'un procédé selon l'une quelconque des revendications précédentes.

**Patentansprüche**

**1.** Verfahren zur Charakterisierung eines Objekts (20), die folgenden Schritte umfassend:

a) Anordnung des Objekts zwischen einer Bestrahlungsquelle (10) und einem Strahlungsdetektor (30), wobei die Bestrahlungsquelle dafür ausgelegt ist, eine ionisierende elektromagnetische Strahlung (12) vom Typ einer Röntgen- oder Gammastrahlung auszusenden, die sich bis zum Objekt ausbreitet;
b) Bestrahlung des Objekts durch die Bestrahlungsquelle (10) und Detektion einer von dem Objekt durchgelassenen Strahlung (14) mithilfe des Strahlungsdetektors, wobei der Strahlungsdetektor mehrere Pixel ($30_i$) aufweist, wobei jedes Pixel ($30_i$) einem Teil ($20_i$) des Objekts zugeordnet ist;

c) für jedes Pixel ($30_i$), Bildung eines Energiespektrums ($S_i$) der detektierten Strahlung, wobei jedes Spektrum wenigstens zwei verschiedene Energiebänder aufweist;

d) aus jedem in Schritt c) gebildeten Spektrum, Schätzung, in jedem Pixel, von wenigstens zwei äquivalenten Dicken ($\hat{L}_{i,1}...\hat{L}_{i,M},\hat{L}'_{i,1}...\hat{L}'_{i,M}$), die jeweils wenigstens zwei Basismaterialien ($mat_1...mat_M,mat'_1...mat'_M$) zugeordnet sind;

wobei das Verfahren **dadurch gekennzeichnet ist, dass** es im Anschluss an Schritt d) die folgenden Schritte umfasst:

e) aus den aus Schritt d) resultierenden äquivalenten Dicken, Berechnung eines strukturellen Parameters des Objekts ($P_i$) in verschiedenen Pixeln ($30_i$), wobei der strukturelle Parameter des Objekts für jedes Pixel:

- eine Dicke ($L_i$) des dem Pixel zugeordneten Teils des Objekts ($20_i$) ist;
- oder für eine Zusammensetzung des dem Pixel zugeordneten Teils des Objekts ($20_i$) repräsentativ ist;

f) räumliche Glättung des in mehreren Pixeln berechneten strukturellen Parameters, um jedem Pixel einen geglätteten strukturellen Parameter $\left( P_i^* \right)$ zuzuordnen;

g) aus dem geglätteten strukturellen Parameter in jedem Pixel und aus jedem in Schritt c) gebildeten Spektrum, Schätzung, in jedem Pixel, von regularisierten äquivalenten Dicken $\left( \tilde{\hat{L}}_{i,m=1}\middle|P_i^* ...\hat{L}_{i,m=M}\middle|P_i^*, \hat{L}'_{i,m=1}\middle|P_i^* ...\hat{L}'_{i,m=M}\middle|\dot{P}_i^* \right),$ die jeweils einem jeweiligen Basismaterial zugeordnet sind;

h) Charakterisierung des Objekts (20) anhand der in Schritt g) geschätzten regularisierten äquivalenten Dicken.

**2.** Verfahren nach Anspruch 1, wobei Schritt d) und/oder Schritt g) für jedes Pixel ($30_i$) eine Berücksichtigung von Kalibrierungsspektren ($S_{cal}(L_1, L_2)$) umfassen, wobei jedes Kalibrierungsspektrum einer Dicke eines jeweiligen Basismaterials zugeordnet ist.

**3.** Verfahren nach Anspruch 2, wobei Schritt d) und/oder Schritt g) außerdem für jedes Pixel ($30_i$) umfassen:

- eine Berechnung einer Likelihood-Funktion ($V_i(S_i, S_{cal}(L_1,L_2)$) aus dem in Schritt c) von dem Pixel gebildeten Spektrum ($S_i$) und aus den Kalibrierungsspektren ($S_{cal}(L_1, L_2)$); wobei jedes Kalibrierungsspektrum wenigstens einem Kalibrierungsmaterial ($mat_1...mat_m...mat_M$) mit einer bekannten Dicke zugeordnet ist;
- eine Bestimmung einer äquivalenten Dicke jedes Kalibrierungsmaterials, welche die Likelihood-Funktion maximiert, wobei jedes Kalibrierungsmaterial ein Basismaterial bildet.

**4.** Verfahren nach Anspruch 3, wobei Schritt d) und/oder Schritt g) einen Basiswechsel zwischen einer Ausgangsbasis, die von den Kalibrierungsmaterialien ($mat_1...mat_m...mat_M$) gebildet wird, und einer Endbasis ($mat'_1...mat'_m...mat'_M$), die von für das Objekt repräsentativen Basismaterialien gebildet wird, umfassen, um eine äquivalente Dicke ($\hat{L}'_{i,1}...\hat{L}'_{i,M}$) jedes Materials der Endbasis zu erhalten.

**5.** Verfahren nach einem der vorhergehenden Ansprüche, wobei Schritt d) umfasst:

- eine Zusammenfassung benachbarter Pixel, um eine Gruppe von Pixeln ($G_j$) zu bilden;
- eine Zuordnung, zu jeder Gruppe von Pixeln, eines zusammengefassten Spektrums ($S_j$), das die für die einzelnen Pixel der Gruppe von Pixeln gebildeten Spektren kombiniert;

derart, dass Schritt d), für wenigstens ein Pixel einer Gruppe von Pixeln, ausgehend von dem zusammengefassten Spektrum durchgeführt wird, das der Gruppe von Pixeln zugeordnet ist.

**6.** Verfahren nach einem der vorhergehenden Ansprüche, wobei der strukturelle Parameter ($P_i$) eine Zusammensetzung des dem Pixel zugeordneten Teils ($20_i$) des Objekts repräsentiert, und wobei der strukturelle Parameter, der in jedem Pixel bestimmt wird:

- eine effektive Ordnungszahl ($Z_{eff,i}$) ist, die aus den in Schritt d) geschätzten äquivalenten Dicken bestimmt wird;
- oder ein Verhältnis einer äquivalenten Dicke eines Basismaterials zur Summe der in Schritt d) geschätzten äquivalenten Dicken ist.

**7.** Verfahren nach einem der vorhergehenden Ansprüche, wobei Schritt h) eine Charakterisierung der verschiedenen Teile ($20_i$) des Objekts umfasst, die verschiedenen Pixeln jeweils zugeordnet sind.

**8.** Verfahren nach Anspruch 7, wobei die Charakterisierung umfasst:

- eine Bildung eines Bildes, das die regularisierte äquivalente Dicke eines Basismaterials zeigt;
- und/oder eine Bestimmung einer effektiven Ordnungszahl ($Z_{eff,i}$) aus den regularisierten äquivalenten Dicken jedes Basismaterials;
- und/oder ein Verhältnis einer regularisierten äquivalenten Dicke eines Basismaterials zur Summe der regularisierten äquivalenten Dicken jedes Basismaterials.

**9.** Verfahren nach einem der vorhergehenden Ansprüche, wobei in Schritt b) der Detektor in Bezug auf das Objekt bewegt wird oder das Objekt in Bezug auf den Detektor bewegt wird.

**10.** Vorrichtung zur Charakterisierung eines Objekts (20), umfassend:

- eine Bestrahlungsquelle (10), die dafür ausgelegt ist, eine ionisierende elektromagnetische Strahlung auszusenden;
- einen Träger (20s), der dazu bestimmt ist, das Objekt aufzunehmen, derart, dass das Objekt zwischen der Bestrahlungsquelle und dem Detektor angeordnet ist;
- einen Detektor (30), der Pixel aufweist, wobei der Detektor dafür ausgelegt ist, eine ionisierende elektromagnetische Strahlung zu detektieren und in mehreren Pixeln ($30_i$) ein Spektrum ($S_i$) der detektierten Strahlung zu bilden;
- einen Prozessor (40), der dafür ausgelegt ist, die von dem Detektor gebildeten Spektren zu empfangen und die Schritte d) bis h) eines Verfahrens nach einem der vorhergehenden Ansprüche durchzuführen.

## Claims

**1.** A method for characterizing an object (20), comprising:

a) placing the object between a radiation source (10) and a radiation detector (30), the radiation source being configured to emit an X or gamma ionizing electromagnetic radiation (12) that propagates to the object;
b) irradiating the object with the radiation source (10) and detecting radiation (14) transmitted by the object using the radiation detector, the radiation detector comprising a plurality of pixels ($30_i$), each pixel ($30_i$)being associated with one portion ($20_i$) of the object;
c) for each pixel ($30_i$), forming an energy spectrum ($S_i$) of the detected radiation, each spectrum comprising at least two distinct energy bands;
d) from each spectrum formed in c), estimating, in each pixel, at least two equivalent thicknesses ($\hat{L}_{i,1}$ ... $\hat{L}_{i,M}, \hat{L}'_{i,1}$ ... $\hat{L}'_{i,M}$) respectively associated with at least two basis materials ($mat_1$ ... $mat_M$, $mat'_1$ ... $mat'_M$);

wherein the method comprises, following d):

e) from the equivalent thicknesses resulting from d), calculating a structural parameter of the object in various pixels, the structural parameter of the object ($P_i$) being for each pixel ($30_i$):

- a thickness ($L_i$) of that portion of the object ($20_i$) which is associated with the pixel;
- or representative of a composition of that portion of the object ($20_i$) which is associated with the pixel.

f) spatially smoothing the structural parameter calculated in a plurality of pixels, so as to associate, with each pixel, a smoothed structural parameter $(P_i^*)$;
g) from the structural parameter smoothed in each pixel, and from each spectrum formed in c), estimating, in each pixel, regularized equivalent thicknesses $(\hat{L}_{i,m=1}|P_i^* ... \hat{L}_{i,m=M}|P_i^*, \hat{L}'_{i,m=1}|P_i^* ... \hat{L}'_{i,m=M}|P_i^*)$ re-

spectively associated with each basis material;

h) characterizing the object (20) from the regularized equivalent thicknesses estimated in g).

2. The method as claimed in claim 1, wherein d) and/or g) comprise(s), for each pixel ($30_i$), taking into account calibration spectra ($S_{cal}(L_1, L_2)$), each calibration spectrum being associated with a thickness of each basis material.

3. The method as claimed in claim 2, wherein d) and/or g) also comprise, for each pixel ($30_i$):

- calculating a likelihood function ($V_i(S_i, S_{cal}(L_1, L_2))$) from the spectrum ($S_i$) formed by the pixel in c) and from the calibration spectra ($S_{cal}(L_1, L_2)$), each calibration spectrum being associated with at least one calibration material ($mat_1 ... mat_m ... mat_M$) of a known thickness;
- determining an equivalent thickness of each calibration material maximizing the likelihood function, each calibration material forming a basis material.

4. The method as claimed in claim 3, wherein d) and/or g) comprise(s) a change of basis, between a input basis, formed by the calibration materials ($mat_1 ... mat_m ... mat_M$), and an output basis ($mat'_1 ... mat'_m ... mat'_M$), formed by the basis materials representative of the object, so as to obtain an equivalent thickness ($\hat{L}'_{i,1} .. \hat{L}'_{i,M}$) of each material of the ouput basis.

5. The method as claimed in any one of the preceding claims, wherein d) comprises:

- grouping adjacent pixels together, in order to form a group of pixels ($G_j$);
- associating, with each group of pixels, a grouped spectrum ($S_j$), combining the spectra formed for each pixel of the group of pixels;

such that d) is implemented, for at least one pixel of a group of pixels, from the grouped spectrum associated with the group of pixels.

6. The method as claimed in in any one of the preceding claims, wherein the structural parameter ($P_i$)represents a composition of that portion ($20_i$) of the object which is associated with each pixel, and wherein the structural parameter, determined in each pixel:

- is an effective atomic number($Z_{eff,i}$), determined from the equivalent thicknesses estimated in d);
- or comprises a ratio between an equivalent thickness of a basis material and the sum of the equivalent thicknesses estimated in d).

7. The method as claimed in any one of the preceding claims, wherein h) comprises a characterization of the various portions ($20_i$) of the object respectively associated with various pixels.

8. The method as claimed in claim 7, wherein the characterization comprises:

- forming an image showing the regularized equivalent thickness of a basis material;
- and/or determining an effective atomic number ($Z_{eff,i}$) from the regularized equivalent thicknesses of each basis material;
- and/or a ratio between a regularized equivalent thickness of a basis material and the sum of the regularized equivalent thicknesses of each basis material.

9. The method as claimed in any one of the preceding claims, wherein, in b), the detector is moved with respect to the object or the object is moved with respect to the detector.

10. A device for characterizing an object, comprising:

- a radiation source (10), configured to emit ionizing electromagnetic radiation;
- a holder (20s), intended to receive the object, such that the object is placed between the radiation source and the detector;
- a detector (30), comprising pixels, the detector being configured to detect ionizing electromagnetic radiation and to form, in a plurality of pixels ($30_i$), a spectrum ($S_i$) of the detected radiation;
- a processor (40), configured to receive the spectra formed by the detector and to implement d) to h) of a method as claimed in any one of the preceding claims.

1

20

20$_i$

10

12

30$_i$

14

20s

40

**Fig. 1A**

$P$

30

1

20

10

12

14

20s

30$_i$

$P$ 30

40

**Fig. 1B**

30

$mat_1$

$mat_2$

$L_1$

$L_2$

**Fig. 1C**

**Fig. 2**

**Fig. 3A**

**Fig. 3B**

**Fig. 3C**

**Fig. 3D**

**Fig. 3E**

**Fig. 3F**

**Fig. 3G**

**Fig. 3H**

**Fig. 3I**

**Fig. 3J**

Fig. 4A                               Fig. 4B

**Fig. 5A**

**Fig. 5B**

**Fig. 5C**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3106864 A

- EP 3084406 A

**Littérature non-brevet citée dans la description**

- **ALVAREZ R.** Near optimal energy selective x-ray imaging system performance with simple detectors. *Med. Phys.,* Février 2010, vol. 37 (2